# EUROPEAN PATENT SPECIFICATION

(11) **EP 3 725 774 B1**
(45) Date of publication and mention of the grant of the patent: **23.02.2022**
(21) Application number: 19460021.9
(22) Date of filing: 17.04.2019
(51) Int. Cl.: C07D 211/58

(54) **PROCESS FOR THE PREPARATION OF A PHARMACEUTICAL AGENT**
VERFAHREN ZUR HERSTELLUNG EINES PHARMAZEUTISCHEN AGENS
PROCÉDÉ DE PRÉPARATION D'UN AGENT PHARMACEUTIQUE

(43) Date of publication of application: 21.10.2020
(73) Proprietor: Zaklady Farmaceutyczne Polpharma S.A., 83-200 Starogard Gdanski (PL)
(72) Inventor: Haluszczuk, Adam, 80-058 Gdansk (PL); Kosik, Kamil, 11-400 Ketrzyn (PL); Piotrkowska, Barbara, 14-520 Pieniezno (PL)
(74) Representative: Rechnio, Justyna Tatiana

(56) References cited:
- WO-A1-2016/141003
- WO-A1-2017/036432
- WO-A2-2008/144326
- CN-A- 107 641 097

## Description

The present invention relates to a process for the preparation of a pharmaceutical agent, and particularly to a process for preparing pimavanserin or an acid addition salt of pimavanserin comprising the following steps: (i) providing an acid addition salt solution of pimavanserin in an organic solvent; (ii) washing the solution provided in step (i) with an alcohol/water/acid mixture;(iii) dissolving the acid addition salt of pimavanserin in an aqueous solvent to form an aqueous solution; (iv) washing the aqueous solution obtained in step (iii) with an organic solvent; (v) adding a base to the washed aqueous solution to form pimavanserin.

Pimavanserin is named 1-[(4-fluorophenyl)methyl]-1-(1-methylpiperidin-4-yl)-3-{[4-(2-methylpropoxy)phenyl]methyl}urea and has the following structure:

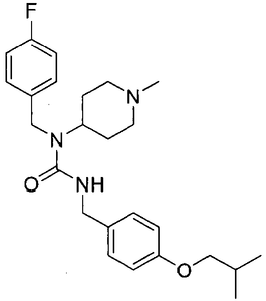

Pimavanserin is an atypical antipsychotic agent which acts as an inverse agonist and antagonist of the serotonin 5-HT_{2A} receptor.

Pimavanserin is marketed in the USA as Nuplazid^{®}. Nuplazid^{®} contains pimavanserin hemitartrate, and is indicated for the treatment of hallucinations and delusions associated with Parkinson's disease psychosis (also known as PDP). Nuplazid^{®} is prescribed as an oral dosage form.

Pimavanserin hemitartrate, 1-(4-fluorobenzyl)-3-(4-isobutoxybenzyl)-1-(1-methylpiperidin-4-yl)urea L-hemitartrate, has the following structure:

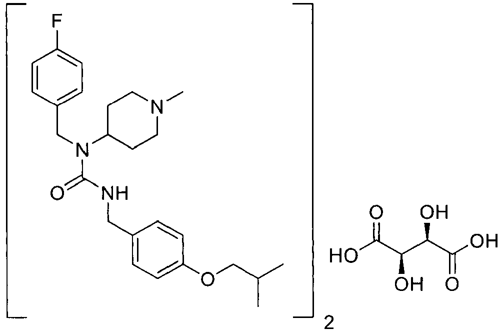

WO 2008/144326 relates to a process for preparing pimavanserin and pimavanserin hemitartrate. The process involves the synthesis of an amine and an isocyanate intermediate, which are then reacted to form pimavanserin:

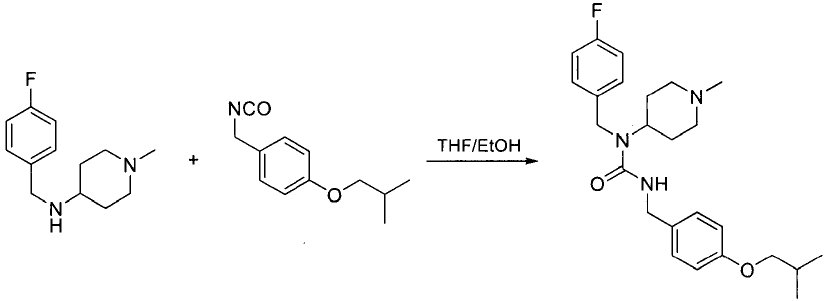

WO 2008/144326 also describes that pimavanserin can be converted into pimavanserin hemitartrate by treatment of the free base with a solution of tartaric acid in ethanol. The preparation of purified pimavanserin and pimavanserin hemitartrate is also described. WO 2008/144326 describes that the purification process requires multiple steps owing to the presence of numerous impurities. Two common impurities are (i) unreacted 4-(4-fluorobenzylamino)-1-methylpiperidine, and (ii) a urea derivative produced by homo-dimerisation of the isocyanate:

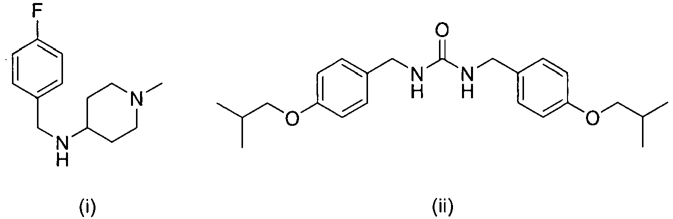

These impurities are described as being particularly difficult to remove, and WO 2008/144326 explains that they must be removed prior to isolation of the final product in order to access pimavanserin or pimavanserin hemitartrate with a high level of purity. To achieve this goal, WO 2008/144326 describes a five-step purification of crude pimavanserin. The steps comprise; (i) formation of a tartaric acid salt; (ii) recrystallisation of the tartaric acid salt; (iii) free-basing of the salt formed in step (ii); (iv) a second formation of a tartaric acid salt; and (v) recrystallisation of the tartaric acid salt.

WO 2016/141003 also relates to a process for preparing pimavanserin and pimavanserin hemitartrate using a range of activated 1-[4-(2-methylpropyloxy)phenyl]methanamine derivatives, in particular 1-[4-(2-methylpropyloxy)phenyl]methanamine activated with 1,1-carbonyldiimidazole and 1-[4-(2-methylpropyloxy)phenyl]methanamine activated with 4-nitrophenyl chloroformate.

Although these processes can provide pimavanserin and pimavanserin hemitartrate with an acceptable yield and purity, they require numerous steps and the isolation of multiple intermediates prior to the isolation of the final product. In particular, the process described in WO 2008/144326 requires the isolation of four separate intermediates prior to isolation of the final product. Consequently, further optimisation of the process is desirable.

Indeed, both large-scale chemical syntheses and purifications are subjected to constant review and optimisation. Owing to economies of scale, any small improvement in a given reaction or purification parameter is particularly economically significant. Therefore, optimisation of a large-scale synthesis or purification that provides, for example, an increase in chemical yield, decrease in step-to-step manipulation, decrease in reaction time, decrease in reaction temperature, decrease in amount of catalyst or solvent used, increase in the favourability of reagents, reduction in side-product formation, a more environmentally benign synthesis or increase in chemical purity is of interest both to chemical manufacturers and suppliers.

Moreover, step optimisation that reduces the need for multiple or hard-to-perform purifications are particularly beneficial. Any improvement in the ease of purification or isolation, through telescoping (wherein two or more, previously independent synthetic transformations converge into a "single" process and therefore require only one purification step) of synthetic procedures, or the identification of an intermediate for recrystallisation, or precipitation, or removal of impurities through conversion into a transient intermediate, or substitution for a cheaper, more environmentally friendly or less toxic reagent, provide an attractive and economically desirable goal. In situations where more traditional purification procedures yield poor or unsuitable results, it becomes necessary that more innovative solutions are required.

However, practical achievement of any such improvement is not straightforward, and even careful optimisation of individual parameters of a synthetic or purification step will often fail to provide a workable advantage within an overall production process.

WO2017/036432 and CN107641097 disclose further processes for the preparation of pimavanserin.

Although processes to prepare pimavanserin are established, there remains a need in the art for improving these processes, in particular with respect to reducing the number of preparation/purification steps, reducing the number of isolated intermediates and increasing the overall yield and purity of the final product.

Accordingly, the present invention provides a process for the preparation of pimavanserin comprising:
(i) providing an acid addition salt solution of pimavanserin in an organic solvent; (ii) washing the solution provided in step (i) with an alcohol/water/acid mixture;(iii) dissolving the acid addition salt of pimavanserin in an aqueous solvent to form an aqueous solution; (iv) washing the aqueous solution obtained in step (iii) with an organic solvent; (v) adding a base to the washed aqueous solution to form pimavanserin.

It has been found that the process provides pure pimavanserin with improved yield and purity.

Accordingly, the present invention also provides a process for the preparation of an acid addition salt of pimavanserin comprising:
(i) providing an acid addition salt solution of pimavanserin in an organic solvent; (ii) washing the solution provided in step (i) with an alcohol/water/acid mixture; (iii) dissolving the acid addition salt of pimavanserin in an aqueous solvent to form an aqueous solution; (iv) washing the aqueous solution obtained in step (iii) with an organic solvent; (v) adding a base to the washed aqueous solution to form pimavanserin; and (vi) converting pimavanserin into an acid addition salt of pimavanserin.

It has been found that the process provides a pure acid addition salt of pimavanserin with improved yield and purity.

In step (i) of the process, an acid addition salt of pimavanserin is provided. The acid addition salt of pimavanserin may be prepared from pimavanserin according to following synthetic protocol (example for pimavanserin hydrochloride shown):

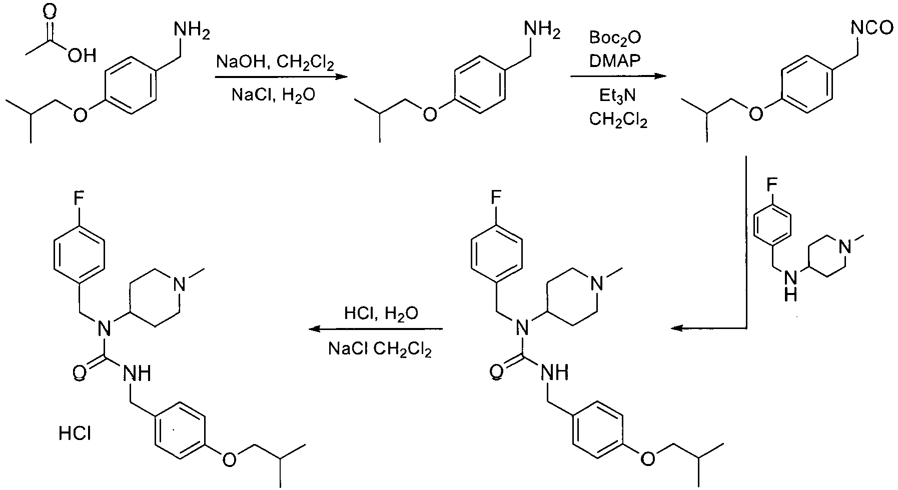

The synthesis begins by conversion of 1-[4-(2-methylpropyloxy)phenyl]methanamine acetate to 1-[4-(2-methylpropyloxy)phenyl]methanamine. The amine is then treated with di-tert-butyl dicarbonate (Boc₂O) in CH₂Cl₂ which provides efficient conversion of the amine to the isocyanate (1-isobutoxy-4-isocyanatomethyl)benzene). The use of di-tert-butyl dicarbonate in this transformation is highly advantageous and circumvents the use of the more toxic and capricious reagent phosgene.

The isocyanate is then reacted with 4-(4-fluorobenzylamino)-1-methylpiperidine to form pimavanserin. 4-(4-fluorobenzylamino)-1-methylpiperidine is accessible by performing a reductive amination upon 1-methyl-4-piperidone with 4-fluorobenzylamine in the presence of a hydride, for example as described in WO 2008/144326.

Preferably, the acid addition salt of pimavanserin provided in step (i) of the process is formed by treating pimavanserin with a mineral acid or an organic acid.

Suitable mineral acids include, hydrochloric acid, sulfuric acid, nitric acid, hydrobromic acid and phosphoric acid; and suitable organic acids include acetic acid, tartaric acid, trifluoroacetic acid, formic acid, oxalic acid, lactic acid, citric acid, methanesulfonic acid and p-toluenesulfonic acid (PTSA). Organic acids typically contain a carboxylic acid group. Organic acids that are mono-carboxylic acid are preferred, however di- and tri-carboxylic acids can also be utilised.

Pimavanserin may be converted into the corresponding hydrochloride salt, by treating pimavanserin with HCI in a mixture of CH₂Cl₂/water/methanol or CHCl₃/water/methanol, or upon treatment with HCl in a mixture of CH₂Cl₂/water or CH₃Cl/water and optionally an additional solvent selected from ethanol, isopropanol, acetone or acetonitrile. Pimavanserin may also be converted into the corresponding hydrogen sulfate, acetate or bitartrate salt upon treatment with sulfuric acid, acetic acid or tartaric acid in the solvent mixtures as described for the conversion of pimavanserin to the corresponding hydrochloride salt.

Where the acid addition salt of pimavanserin is provided by this process, the salt is typically provided as a solution in an organic solvent, for example in a solution of CH₂Cl₂ or CH₃Cl. The solution is provided by separating the organic solvent phase from the aqueous solvent phase. The acid addition salt of pimavanserin may be retained in solution within the organic solvent for further use. The organic solvent may also be removed, for example by distillation to yield a pimavanserin acid addition salt residue for further use.

Alternatively, pimavanserin can be sourced commercially and converted directly into an acid addition salt of pimavanserin in a single step using the conditions defined hereinabove.

Preferably, the acid addition salt of pimavanserin provided in step (i) of the process is pimavanserin hydrochloride, pimavanserin hydrogen sulfate or pimavanserin acetate. The most preferred acid addition salt of pimavanserin provided in step (i) of the process is pimavanserin hydrochloride.

In step (iii) of the process the acid addition salt of pimavanserin is dissolved in an aqueous solvent to form an aqueous solution. The aqueous solvent is preferably water or a mixture of water and an alcohol. Where the aqueous solvent contains a mixture of water and an alcohol, the amount of alcohol will not exceed 50% of the total amount of solvent in the mixture. Where an alcohol is present, the alcohol is preferably methanol. The aqueous solvent, whether used alone or as a mixture with an alcohol may also contain sodium chloride.

In step **(iv)** of the process, the aqueous solution obtained in step (iii) is subjected to a washing step with an organic solvent.

In this step, the aqueous solution obtained in step (iii) is washed with an organic solvent and the organic solvent is discarded.

In this step the acid addition salt of pimavanserin is not converted to pimavanserin (i.e. pimavanserin free base) during the washing step. Instead, the acid addition salt of pimavanserin is retained as such throughout the washing step. This is advantageous, as during the washing step, the acid addition salt of pimavanserin is retained in the aqueous solvent.

The washing (or extraction) of aqueous solutions with organic solvents is a standard technique in the art which takes advantage of the solubility of different substances in aqueous and organic solvents and the immiscibility of aqueous/organic solvents which allows for separation of the solvents and thus the substances contained therein.

Miscibility is the property of two substances to mix in all proportions, that is, to fully dissolve in each other at any concentration to form a homogenous solution. For example, water and ethanol are miscible because they mix in all proportions. Conversely, substances are said to be immiscible if there are certain proportions in which the mixture does not form a homogenous solution. Miscibility of two substances is often determined optically, for example when the two miscible liquids are combined the resulting liquid is clear. Likewise if upon mixing two substances two phases form (for example an aqueous phase and an organic phase) the two substances are also said to be immiscible. On the other hand, if two immiscible liquids are combined, the resulting liquid is cloudy and no phase separation occurs. That is, where an organic solvent and water are mixed, the mixture is said to be immiscible when either two phases form or an emulsion is observed. In situations where emulsions form, it is known that phase formation and solvent separation may be improved by the addition of solid sodium chloride or a solution of sodium chloride.

The washing step is advantageous, because the use of an organic solvent as the washing solvent allows organic impurities present within the aqueous solution obtained in step (ii) or (iii) of the process to partition from the aqueous phase into the organic phase. The partitioning of the organic impurities is driven by the inherent solubility difference of the impurities in the two phases. Partitioning of the impurities into the organic phase provides purification of the aqueous phase, and thus the acid addition salt of pimavanserin which is advantageously retained in the aqueous phase during the washing step owing to its increased solubility in water versus the organic solvent. If required, sodium chloride can be added to the aqueous phase during the washing step to improve phase formation and thus separation of the aqueous and organic phases.

In step (iv), the organic solvent therefore acts as a washing solvent to wash the aqueous solution.

Preferably, the organic solvent is a water-immiscible organic solvent.

The organic solvent may comprise a water-immiscible organic solvent for example methyl-t-butyl ether, Me-THF, pentane, hexane, heptane, cyclohexane, methoxycyclohexane, diethyl ether, diisopropyl ether, ethyl acetate, isopropyl acetate, butyl acetate, methyl ethyl ketone, toluene, xylenes or cyclopentyl methyl ether. The most preferred organic solvent is methyl-t-butyl ether.

In one embodiment the washing step comprises addition of the organic solvent to the aqueous solution obtained in step (iii) and stirring of the mixture, for example for 1 to 60 minutes, before the aqueous phase and the organic solvent phase are allowed to separate, and the organic solvent is discarded. Most preferably, the mixture is stirred for 30 minutes before the phases are allowed to separate and the organic solvent is discarded.

The washing step may comprise addition of the organic solvent to the aqueous solution obtained in step (iii) and stirring of the mixture, for example for 1 to 60 minutes, preferably 30 minutes wherein the temperature of the mixture is 20-45°C. It is most preferred when the mixture is stirred for 30 minutes, and the temperature of the mixture is 35-40°C.

The washing step may be performed once or multiple times depending upon the scale of the reaction i.e. the amount of the acid addition salt of pimavanserin that requires purification. Throughout the washing step, the aqueous solution is retained because it contains the acid addition salt of pimavanserin.

The skilled person will be aware of how to assess when the washing step is complete. For example, the skilled person could take an aliquot of the organic solvent used to wash the aqueous phase, and check if any further impurity was present by conducting TLC or HNMR analysis. Washing is typically deemed complete when no impurities are detectable in the organic solvent.

In step (v) of the process a base is added to the washed aqueous solution containing the acid addition salt of pimavanserin to form pimavanserin. Typically, the base is dissolved or suspended in water, and then added as solution or suspension to the washed aqueous solution containing the acid addition salt of pimavanserin.

In one embodiment, the base is a metal carbonate or a metal hydroxide. Preferably the base is a metal carbonate.

The base may comprise a metal carbonate selected from lithium, sodium, potassium or ammonium carbonate, or a metal hydroxide selected from lithium, sodium, potassium, magnesium or calcium hydroxide. Preferably the base is sodium hydrogen carbonate (i.e. sodium bicarbonate) or sodium hydroxide.

Typically, the base is added to the washed aqueous solution containing the acid addition salt of pimavanserin until the pH of the aqueous solution is close to, or just basic. Preferably, the base is added until the pH of the aqueous solution is 6.0-14.0, more preferably 6.5-14.0. Most preferably the base is added until the pH of the aqueous solution is 6.5-8.0 or 7.0-8.0.

In one embodiment the base is sodium hydrogen carbonate and the sodium hydrogen carbonate is added until the pH of the aqueous solution is 6.5-8.0 or 7.0-8.0.

The base may be added to the washed aqueous solution containing the acid addition salt of pimavanserin and the mixture be stirred, for example for 1 to 60 minutes. Most preferably, the mixture is stirred for 30 minutes. Optionally the base may be added to the aqueous solution containing the acid addition salt of pimavanserin and the mixture be stirred, for example for 1 to 60 minutes, wherein the temperature of the mixture is 20-45°C. It is most preferred when the mixture is stirred for 30 minutes, and the temperature of the mixture is 35-40°C. Optionally, toluene may be added to the washed aqueous solution containing the acid addition salt of pimavanserin before the addition of the base. After addition of the base, the pH of the aqueous solution can be measured using universal indicator paper or a pH meter. Once the aqueous solution is confirmed to have a basic pH, pimavanserin can be extracted from the aqueous phase using an organic solvent, for example toluene, xylene, methyl-t-butyl ether, ethyl acetate, isopropyl acetate, Me-THF or cyclopentyl methyl ether. The most preferred solvent for extracting pimavanserin base is toluene.

Pimavanserin is preferably extracted into the organic solvent at a temperature of 20-45°C, most preferably 35-40°C.

Pimavanserin can be isolated from the organic solvent by filtration or removal of the solvent, for example by evaporation of the solvent.

In one embodiment, the pimavanserin obtained in step (v) is isolated and further purified by recrystallisation. Typically, the recrystallisation of pimavanserin is performed by dissolving pimavanserin in an organic solvent and cooling the solution. Preferably, the solution is cooled to 0-5°C.

The recrystallisation of pimavanserin may be performed by dissolving pimavanserin in a mixture of a solvent and an anti-solvent. For example, wherein the solvent is selected from toluene, xylene, methyl-t-butyl ether, ethyl acetate, isopropyl acetate, Me-THF or cyclopentyl methyl ether; and the anti-solvent is selected from petroleum ether, pentane, hexane, heptane, cyclohexane and methoxycyclohexane or mixtures thereof. Preferably, the anti-solvent is heptane. The heptane may be added to a solution of pimavanserin in the first solvent, and the resulting mixture cooled to 0-5°C.

Following recrystallisation pimavanserin is typically collected by filtration and dried. Prior to drying the recrystallised product, pimavanserin may be washed with a mixture of a solvent and anti-solvent. For example, wherein the solvent is selected from toluene, xylene, methyl-t-butyl ether, ethyl acetate, isopropyl acetate, Me-THF or cyclopentyl methyl ether; and the anti-solvent is selected from petroleum ether, pentane, hexane, heptane, cyclohexane and methoxycyclohexane or mixtures thereof. Preferably, pimavanserin may be washed with heptane.

In one embodiment, the present invention provides a process for the preparation of an acid addition salt of pimavanserin.

The process for preparing the acid addition salt of pimavanserin comprises steps (i)-(v) as defined hereinabove and additionally step (vi).

In step (vi) pimavanserin is converted into an acid addition salt of pimavanserin, preferably a pharmaceutically acceptable acid addition salt. The pharmaceutically acceptable acid addition salt obtained by performing step (vi) may be the same salt, or may be a different salt to the acid addition salt of pimavanserin provided in step (i) of the process.

The term "pharmaceutically acceptable salt" refers to a salt of a compound that does not cause significant irritation to an organism to which it is administered. Preferred pharmaceutically acceptable salts can be selected from hydrochloride, hydrobromide, hydrogen sulfate, phosphate, aliphatic or aromatic carboxylic or sulfonic acids, for example tartaric, acetic, succinic, lactic, malic, citric, ascorbic, nicotinic, methanesulfonic, ethanesulfonic, p-toluensulfonic, salicylic or naphthalenesulfonic acid. The skilled person is well-equipped to determine the necessary conditions required to perform a salt formation reaction, and any such method contained within his common general knowledge is envisaged as being suitable.

Advantageously, the process provides a pure acid addition salt of pimavanserin from pimavanserin in a single step, requiring the isolation of only one intermediate in addition to the final acid addition salt of pimavanserin product.

Preferably, in step (vi) of the process, pimavanserin is converted into pimavanserin hemitartrate.

Typically, pimavanserin is converted into pimavanserin hemitartrate by treating a solution of pimavanserin with a solution of tartaric acid. For example, a solution of pimavanserin in ethanol is treated with a solution of tartaric acid in ethanol.

Prior to step (vi) the pimavanserin obtained in step (v) of the process may be dried to remove any residual water. Preferably, the pimavanserin is dried in a tray dryer, for example at a temperature above 50°C, for example at 50-55°C.

Pimavanserin may be converted to pimavanserin hemitartrate by treating a solution of pimavanserin in a solvent mixture, wherein the first solvent is selected from acetone and methyl-ethyl-ketone; and the second solvent is selected from methyl-t-butyl ether, THF, Me-THF and heptane, with a solution of tartaric acid in acetone or methyl-ethyl-ketone. Preferably, pimavanserin is dissolved in a mixture of methyl-t-butyl ether and acetone and treated with a solution of tartaric acid in acetone.

Pimavanserin may be dissolved in a solvent mixture, wherein the first solvent is selected from acetone and methyl-ethyl-ketone; and the second solvent is selected from methyl-t-butyl ether, THF, Me-THF and heptane. Preferably, pimavanserin is dissolved in a mixture of methyl-t-butyl ether and acetone. Commercially available solvents are suitable for dissolving pimavanserin, and solvents having a residual water content of less than 0.5% are preferred. It has been found that the use of solvents with low residual water content further increase the yield of conversion of pimavanserin to pimavanserin hemitartrate.

In one embodiment, prior to converting pimavanserin into pimavanserin hemitartrate in step (vi) the process comprises the step of dissolving pimavanserin in a solvent to form a solution and optionally adding a seed of pimavanserin hemitartrate form C thereto. Preferably, a seed of pimavanserin hemitartrate form C may be added to the solution of pimavanserin, and the resulting mixture heated to reflux. Following addition of the seed of pimavanserin hemitartrate form C, a solution of tartaric acid, preferably a solution of tartaric acid in acetone or methyl-t-butyl ether, is added to the solution containing pimavanserin and the seed of pimavanserin hemitartrate form C, and heating at reflux temperature is maintained.

Preferably, pimavanserin and tartaric acid are then allowed to react together in the presence of the seed of pimavanserin hemitartrate form C. Preferably, the reaction is conducted at reflux. Preferably, the pimavanserin hemitartrate obtained in step (vi) of the process is pimavanserin hemitartrate form C.

Advantageously, this step converts pimavanserin to pimavanserin hemitartrate form C. Without wishing to be bound by theory, it is thought that the addition of the seed of pimavanserin hemitartrate form C prior to treating pimavanserin with a solution of tartaric acid in step (vi) assists the formation of pimavanserin hemitartrate form C as the final product.

In one embodiment, pimavanserin and tartaric acid are allowed to react together in the absence of a seed of pimavanserin hemitartrate form C. Preferably, the reaction is conducted at reflux. Preferably, the pimavanserin hemitartrate obtained in step (v) of the process is pimavanserin hemitartrate.

WO 2008/144326 describes obtaining pimavanserin hemitartrate in various other crystalline forms (forms A-F) by reference to characteristic X-ray powder diffractograms, the characterising peaks of which are reported therein.

Form C is the preferred polymorphic form of pimavanserin hemitartrate owing to its increased chemical and physical stability over other known forms. Form C is also particularly advantageous for processing and handling pimavanserin hemitartrate in multi-kilogram quantities.

As reported by WO 2008/144326, the X-ray powder diffractogram of pimavanserin hemitartrate form C has characteristic peaks as follows.

XRPD 2θ (°): 7.3, 8.2, 11.9, 12.8, 13.5, 14.3, 15.1, 16.0, 16.8, 17.2, 18.3, 18.9, 19.4, 20.3, 21.7, 22.5, 23.6, 24.0, 25.5, 25.7, 26.1, 27.5, 29.0 and 30.5.

Seed material of pimavanserin hemitartrate form C can be prepared as described in WO 2008/144326.

In a further embodiment of the processes defined hereinabove, the processes may comprise an additional step, wherein 1-[4-(2-methylpropyloxy)phenyl]methanamine acetate is purified by recrystallisation, and the recrystallised 1-[4-(2-methylpropyloxy)phenyl]methanamine acetate is used to prepare the acid addition salt of pimavanserin provided in step (i) of the processes.

Preparation of the acid addition salt of pimavanserin using recrystallised 1-[4-(2-methylpropyloxy)phenyl]methanamine acetate is advantageous, because the substance is known to contain isomeric impurities in the form of 1-[4-(1-methylpropyloxy)phenyl]methanamine acetate (sec) and 1-[4-butyloxyphenyl]methanamine acetate (butyl) which have the following structures:

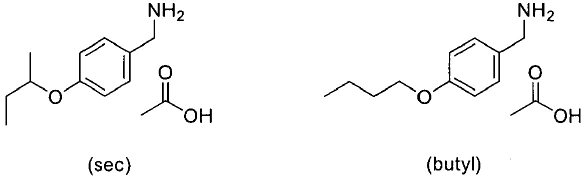

Failure to remove these isomeric impurities can lead to the formation of the corresponding unwanted acid addition salts of pimavanserin (and so pimavanserin) isomers downstream in the synthetic sequence:

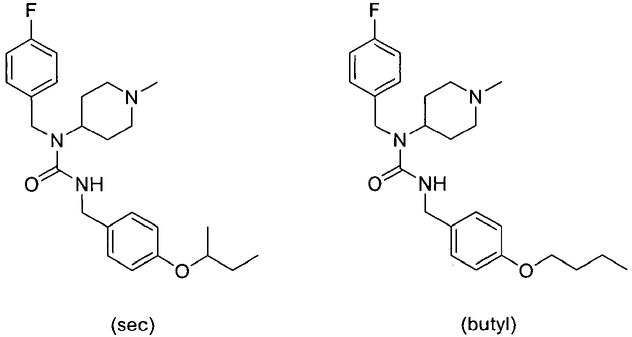

Advantageously, recrystallisation of 1-[4-(2-methylpropyloxy)phenyl]methanamine acetate allows for the removal of both 1-[4-(1-methylpropyloxy)phenyl]methanamine acetate (sec) and 1-[4-(butyloxy)phenyl]methanamine acetate (butyl) impurities, which in turn prevents the formation of the corresponding unwanted and difficult to remove acid addition salts of pimavanserin (and so pimavanserin) isomers in the process of the present invention.

The 1-[4-(2-methylpropyloxy)phenyl]methanamine acetate may be recrystallised from an alcoholic solvent, or a mixture of an alcoholic solvent and a non-alcoholic solvent. Preferably, 1-[4-(2-methylpropyloxy)phenyl]methanamine acetate is recrystallised from isopropyl alcohol or from a mixture of methanol and methyl-t-butyl ether. Most preferably, 1-[4-(2-methylpropyloxy)phenyl]methanamine acetate is suspended in MeOH or in a mixture of MeOH and methyl-t-butyl ether and heated to 50-60°C to dissolve the suspension, and the recrystallised product is collected by filtration.

In a further preferred embodiment 1-[4-(2-methylpropyloxy)phenyl]methanamine acetate is suspended in a mixture of MeOH and methyl-t-butyl ether, the mixture is heated to 50-60°C (i.e. to reflux) to dissolve the suspension, further methyl-t-butyl ether is added, heating under reflux is continued before the solution is allowed to cool, the solution is then further cooled to 0-5°C and the recrystallised product is collected by filtration.

Advantageously, the acid addition salt of pimavanserin can be prepared by reacting 1-(isocyanatomethyl)-4-(2-methylpropoxy)benzene with N-(4-fluorobenzyl)-1-methylpiperidin-4-amine wherein 1-(isocyanatomethyl)-4-(2-methylpropoxy)benzene is prepared from 1-[4-(2-methylpropyloxy)phenyl]methanamine acetate that has been recrystallised from an alcoholic solvent, or a mixture of an alcoholic solvent and a non-alcoholic solvent, preferably from isopropyl alcohol or from a mixture of methanol and methyl-t-butyl ether. This process provides the acid addition salt of pimavanserin with increased purity.

In one embodiment, the present invention provides a process for the preparation of pimavanserin or an acid addition salt thereof comprising an additional step performed before step (i) of reacting 1-(isocyanatomethyl)-4-(2-methylpropoxy)benzene with N-(4-fluorobenzyl)-1 -methylpiperidin-4-amine to form pimavanserin. Preferably, the 1-(isocyanatomethyl)-4-(2-methylpropoxy)benzene is prepared from 1-[4-(2-methylpropyloxy)phenyl]methanamine acetate that has been recrystallised from an alcoholic solvent, or a mixture of an alcoholic solvent and a non-alcoholic solvent.

The present invention also provides a process for the preparation of pimavanserin or an acid addition salt of pimavanserin comprising:
(i) providing an acid addition salt solution of pimavanserin in an organic solvent;
(ii) washing the solution provided in step (i) with an alcohol/water/acid mixture;
(iii) dissolving the acid addition salt of pimavanserin in an aqueous solvent to form an aqueous solution;
(iv) washing the aqueous solution obtained in step (iii) with an organic solvent;
(v) adding a base to the washed aqueous solution to form pimavanserin; and optionally
(vi) converting pimavanserin into an acid addition salt of pimavanserin.

Preferably in step (i) the acid addition salt of pimavanserin is provided in an organic solvent, for example in dichloromethane or chloroform.

Preferably, the acid addition salt of pimavanserin provided in step (i) of the process is formed by treating pimavanserin with a mineral acid or an organic acid as described hereinabove.

Preferably, the acid addition salt of pimavanserin provided in step (i) of the process is pimavanserin hydrochloride, pimavanserin hydrogen sulfate or pimavanserin acetate. The most preferred acid addition salt of pimavanserin provided in step (i) of the process is pimavanserin hydrochloride.

Preferably in step (ii) the solution provided in step (i) is washed with an alcohol/water/acid mixture, for example a mixture of MeOH/water/hydrochloric acid or a mixture of MeOH/water/sulfuric acid or a mixture of MeOH/water/acetic acid. Advantageously this step allows for the removal of impurities from the organic solvent in the respective alcohol/water/acid mixture whilst the pimavanserin acid addition salt is retained in the organic solvent. In this step the respective alcohol/water/acid mixture is discarded, and the organic solvent solution is retained.

Preferably steps (iii)-(vi) are performed as defined hereinabove.

Accordingly, the present invention also provides a process for preparing a dosage form comprising pimavanserin, comprising the steps of preparing pimavanserin according to the invention and combining pimavanserin with one or more pharmaceutically acceptable excipients.

Accordingly, the present invention also provides a process for preparing a dosage form comprising an acid addition salt of pimavanserin, comprising the steps of preparing an acid addition salt of pimavanserin according to the invention and combining the acid addition salt of pimavanserin with one or more pharmaceutically acceptable excipients.

Preferably, the present invention also provides a process for preparing a dosage form comprising pimavanserin hemitartate, comprising the steps of preparing pimavanserin hemitartate according to the invention and combining pimavanserin hemitartate with one or more pharmaceutically acceptable excipients.

Pimavanserin hemitartrate is typically administered orally and so the dosage form is preferably an oral dosage form, most preferably a tablet. In such a case, the process further comprises a tableting step.

The oral dosage form may be a coated or uncoated tablet and may be prepared using standard techniques known in the art.

Where the oral dosage form is a coated tablet, the tablet is preferably a film-coated tablet. Typically, the film-coated tablet will contain 20 mg of pimavanserin hemitartrate, which is equivalent to 17 mg of pimavanserin free base. Excipients formulated with the active ingredient typically include pregelatinised starch, magnesium stearate, and microcrystalline cellulose. Additionally, the film-coat is typically formulated with excipients which include hypromellose, talc, titanium dioxide, polyethylene glycol, and saccharin sodium.

The oral dosage form may also be provided as a capsule. For example a capsule containing 34 mg of pimavanserin hemitartrate.

Also disclosed, but not part of the invention is pimavanserin obtainable by the process of the invention, an acid addition salt of pimavanserin obtainable by the process of the invention, and a pimavanserin dosage form comprising pimavanserin or an acid addition salt of pimavanserin obtainable by the process of the invention.

The products obtainable by the processes are different to the products obtainable by the processes of the prior art, as demonstrated by the data showing that pimavanserin and pimavanserin hemitartrate have improved purity relative to the products obtained by the prior art. Indeed, pimavanserin is obtained with HPLC purity beyond 99.5%, and subsequent transformation to pimavanserin hemitartrate provide the hemitartrate salt with no detectable impurities.

Also disclosed, but not part of the invention is the use of pimavanserin hydrochloride, pimavanserin hydrogen sulfate or pimavanserin acetate for preparing pimavanserin or an acid addition salt thereof.

Also disclosed, but not part of the invention is the use of pimavanserin hydrochloride, pimavanserin hydrogen sulfate, or pimavanserin acetate for preparing pimavanserin hemitartate.

Advantageously, pimavanserin hydrochloride, pimavanserin hydrogen sulfate and pimavanserin acetate are more easily purified than crude pimavanserin, and thus allow for the provision of any other so desired pimavanserin form, for example the free base upon treatment of the hydrochloride, hydrogen sulfate or acetate salt with a base, or any other acid addition salt thereof upon treatment of the hydrochloride, hydrogen sulfate, or acetate salt with a base followed by conversion of the base to an acid addition salt. Furthermore the pimavanserin or any other acid addition salt form thereof prepared by the process of the present invention is provided in improved yield and purity compared to the prior art.

The present invention will now be described with reference to the following examples, which are not intended to be limiting.

### Examples

### Example 1: Preparation of pimavanserin hydrochloride

Pimavanserin free base was added to CH₂Cl₂ and a solution containing water (180 mL), HCl (62.5 mL) (32-38%), 5 g NaCl and methanol (50 mL) was added. The obtained mixture was heated to 25-30°C and stirred for 1 hour, after which the phases were allowed to separate and the aqueous phase was discarded. A second solution containing water (180 mL), HCl (62.5 mL) (32-38%), 5 g NaCl and methanol (50 mL) was then added to the organic phase. After stirring for 1 hour the phases were separated and the aqueous phase was discarded. A third solution containing water (100 mL), methanol (20 mL) and 5 g NaCl was then added to the organic phase. The reaction mixture was stirred at 25-30°C for 30 minutes and the phases were separated. A fourth solution containing water (100 mL), methanol (20 mL) and 5 g NaCl was then added to the organic phase. The reaction mixture was stirred for 30 minutes at 25-30°C after which the phases were allowed to separate and the aqueous phase was discarded. The organic phase was then heated to reflux and the solvents were distilled out at normal (i.e. atmospheric) pressure to obtain pimavanserin hydrochloride as an oily residue.

### Example 2: Preparation and purification of pimavanserin

Pimavanserin hydrochloride was dissolved in a mixture of water (500 mL), methanol (50 mL) and 2.5 g NaCl, and the temperature of the mixture was adjusted to 35-40°C. Methyl-t-butyl ether (MTBE) (250 mL) was added and the reaction mixture was stirred for 30 minutes at 35-40°C, the phases were allowed to separate and the organic phase was discarded. MTBE (250 mL) was then added to the aqueous phase (containing pimavanserin hydrochloride) and stirring at 35-40°C was continued for 30 minutes, after which the phases were allowed to separate and the organic phase was discarded. Toluene (360 mL) was then added to the aqueous phase, followed by a solution of 8% NaHCO₃ which was added until the pH of the solution reached 7.0-7.8. After stirring for 30 minutes at 35-40°C the phases were allowed to separate and the aqueous phase was discarded. Water (150 mL) and n-heptane (200 mL) were then added to the organic phase at 35-40°C. The reaction mixture was stirred for 30 minutes, the phases were separated and the aqueous phase was discarded. n-heptane (400 mL) was then added dropwise to the organic phase at 35-40°C. The obtained reaction mixture was stirred for 3 hours at 35-40°C, cooled to 0-5°C over a period 5 hours, and stirred at 0-5°C for 4 hours. The product was then filtered and washed, first with a mixture of toluene:n-heptane and then with n-heptane and to obtain pimavanserin free base.

Pimavanserin was obtained 39.1 g (80%, 0.091 mol) (36 g, 0.084 mol after additional crystallisation) with a HPLC purity above 99.50% over two steps.

Optionally, the free base may be further purified, whereby pimavanserin free base was then dissolved in toluene (280 mL) at 35-40°C. Water (120 mL) and n-heptane (150 mL) were then added. The reaction mixture was stirred at 35-40°C for 30 minutes, the phases were separated and the aqueous phase was discarded. n-heptane (310 mL) was then added dropwise to the organic phase at 35-40°C. The obtained reaction mixture was stirred for 3 hours at 35-40°C, cooled to 0-5°C over 5 hours and then stirred for 4 hours at 0-5°C. The product was filtered and washed, with a mixture of toluene:n-heptane and then with n-heptane. The product was then dried at 50-55°C in a tray dryer.

### Example 3: Preparation and purification of pimavanserin hemitartrate

Pimavanserin base (10 g, 0.0234 mol) was dissolved in a mixture of acetone (40 mL) and MTBE (100 mL) at 35-40°C. The obtained solution was filtered and a seed of pure pimavanserin hemitartrate form C (0.05 g) was added and the solution was heated to reflux (50-55°C). After 1 hour at reflux, a solution of (1.75 g, 0.0117 mol) L-tartaric acid in acetone (60 mL) was added dropwise. The obtained suspension was stirred at reflux for 1 hour, cooled to 20-25°C over 4 hours and stirred at 20-25°C for 4 hours. The product was filtered off, washed with a mixture of acetone:MTBE (20 mL) and with MTBE (20 mL). After drying at 50-55°C in a vacuum dryer 11.17 g (95%, 0.0222 mol) of pimavanserin hemitartrate form C was obtained with a HPLC purity above 99.75%.

**Table 1.**

| | Comparative example, not of the invention* | Process according to the invention |
|---|---|---|
| Yield** | 65% | 76% |
| Impurities*** | 0.2% | No trace |
| Isolation steps | 4 isolated intermediates + isolation of pimavanserin hemitartrate | 1 isolated intermediate + isolation of pimavanserin hemitartrate |

| | | |
|---|---|---|
| * WO 2008/144326 **Calculated from pimavanserin free base prior to conversion to the hydrochloride ***Amount of N-(4-fluorobenzyl)-1-methylpiperidin-4-amine in final product | | |

### Example 3A: Preparation and purification of pimavanserin hemitartrate

Pimavanserin base 7 g (164 mmol) was dissolved in a mixture of acetone (28 mL) and heptane (70 mL) at 35-40°C. The obtained solution was filtered, and a seed of pure pimavanserin hemitartrate form C (0.04 g) was added and the solution was heated to reflux (50-55°C). A solution of 1.23 g (82 mmol) of L-tartaric acid in acetone (42 mL) was then added over 1 hour. The obtained suspension was stirred at reflux for 1 hour, cooled to 20-25°C over 4 hours and stirred at 20-25°C for 4 hours. The product was filtered, washed with a mixture of acetone:heptane (15 mL) and with heptane (15 mL). After drying at 50-55°C in a vacuum dryer 8.18 g (99%) of pimavanserin hemitartrate form C was obtained with a HPLC purity of 99.76%.

### Example 3B: Preparation and purification of pimavanserin hemitartrate

Pimavanserin base 7 g (164 mmol) was dissolved in a mixture of methyl ethyl ketone (MEK) (28 mL) and heptane (70 mL) at 35-40°C. The obtained solution was filtered, and a seed of pure pimavanserin hemitartrate form C (0.04 g) was added and the solution was heated to reflux (50-55°C). A slurry of 1.23 g (82 mmol) of L-tartaric acid in MEK (42 mL) was then added over 5 minutes. The obtained suspension was stirred at reflux for 1 hour, cooled to 20-25°C over 4 hours and stirred at 20-25°C for 4 hours. The product was filtered, washed with a mixture of MEK:MTBE (15 mL) and with MTBE (15 mL). After drying at 50-55°C in a vacuum dryer 7.41 g (90%) of pimavanserin hemitartrate form C was obtained with a HPLC purity of 99.79%.

### Example 4: Preparation and purification of pimavanserin in "one pot" proceeding through pimavanserin hydrochloride (without isolation)

BOC₂O (7.40 g, 33.9 mmol) and DMAP (0.68 g, 5.56 mmol) were dissolved in dichloromethane (34 mL) at 20°C. The reactor content was cooled to -15°C, and a solution of 1-[4-(2-methylpropyloxy)phenyl]methanamine (5.05 g, 28.2 mmol) in mixture of dichloromethane (150mL) and triethylamine (4 mL) was added dropwise to the reaction mixture over 40-90 minutes keeping internal temperature below -5°C. After complete addition the reaction mass was stirred for 1 hour at -5 to 5°C, before 4-(4-fluorobenzylamino)-1-methylpiperidine (6.88 g, 31.0 mmol) was added. The reaction mixture was then heated to 0-5°C and stirred at this temperature for 2 hours. NaOH (10% solution) (62 mL) was then added dropwise whilst keeping the reaction temperature below 10°C. After stirring for 6 hours at 0-5°C, stirring was stopped and phases were separated.

To the organic phase was added a solution containing water (45 mL), 32-38% HCI (15.5 mL), 1.25 g of NaCl and methanol (12.5 mL). The obtained reaction mixture was heated to 25-30°C and stirred for 1 hour. Pimavanserin hydrochloride was obtained as a solution in dichloromethane, but was not isolated. After phase separation (aqueous phase was discarded), water (45 mL), 32-38% HCl (15.5 mL), 1.25 g of NaCl and methanol (12.5 mL) were added to the organic phase. After stirring for 1 hour the phases were separated and the aqueous phase was discarded, and then a solution containing water (25 mL), methanol (5 mL) and 1.25 g of NaCl was added to the organic phase. The reaction mixture was stirred at 25-30°C for 30 minutes and the phases were separated, and again, a solution containing water (25 mL), methanol (5 mL) and 1.25 g of NaCl was added to the organic phase and the reaction mixture was stirred at 25-30°C for 30 minutes and the phases were separated and the aqueous phase was discarded. The organic phase was heated to reflux and solvents were distilled out at normal pressure to obtain pimavanserin hydrochloride as an oily residue.

To residual pimavanserin hydrochloride was added water (125 mL), methanol (12.5 mL) and 0.5 g of NaCl. After dissolution, the temperature of the reaction mixture was adjusted to 35-40°C, and then n-heptane (62 mL) was added and the mixture was stirred for 30 minutes at 35-40°C. The phases were then separated and the organic phase was discarded. To the aqueous phase (containing pimavanserin hydrochloride) n-heptane (62 mL) was added and the mixture was stirred at 35-40°C for 30 minutes. The phases were separated and the organic phase was discarded. Then, to the aqueous phase toluene (90 mL) was added. An 8% solution of NaHCO₃ was added dropwise to the aqueous/toluene solution until a pH value of 7.0-7.8 was reached. After stirring for 30 minutes at 35-40°C phases were separated, the aqueous phase was discarded and the organic phase (containing pimavanserin base) was used in next extraction. To organic phase was added water (37 mL) and n-heptane (50 mL) at 35-40°C. The reaction mixture was stirred for 30 minutes, the phases were separated and the aqueous phase was discarded. To the organic phase, n-heptane (100 mL) was added dropwise at 35-40°C. The obtained reaction mixture was stirred for 3 hours at 35-40°C, then cooled to 0-5°C over 5 hours, and stirred at 0-5°C for 4 hours. The product was filtered, washed first with a mixture of toluene:n-heptane and then with n-heptane.

The product was dried at 50-55°C to yield 8.69 g (72%) of pimavanserin base with a HPLC purity of 99.52% over three steps.

### Example 5: Preparation and purification of pimavanserin in "one pot" proceeding through pimavanserin hydrogen sulfate

Boc₂O (7.40 g, 33.9 mmol) and DMAP (0.68 g, 5.56 mmol) were dissolved in dichloromethane (34 ml) at 20°C. The reactor content was cooled down to -15°C, and a solution of 1-[4-(2-methylpropyloxy)phenyl]methanamine (5.05 g, 28,2 mmol) in mixture of dichloromethane (150 mL) and trimethylamine (4 mL) was added dropwise to the reaction mixture within 40-90 minutes keeping internal temperature below -5°C. After complete addition the reaction mass was stirred for 1 hour at 15°C, and 4-(4-fluorobenzylamino)-1-methylpiperidine (6.88 g, 31.0 mmol) was added. The reaction mixture was then heated to 0-5°C and stirred at this temperature for 2 hours. NaOH (10% solution) (62 mL) was then added dropwise whilst keeping the reaction temperature below 10°C. After stirring for 6 hours at 0-5°C, stirring was stopped and phases were separated.

To the organic phase (dichloromethane) was added a solution containing water (45 mL), conc. H₂SO₄ (9.9 mL), 1.25 g of NaCl and methanol (12.5 mL). The obtained reaction mixture was heated to 25-30°C and stirred for 1 hour. Pimavanserin hydrogen sulfate was obtained as a solution in dichloromethane, but was not isolated. After phase separation (aqueous phase was discarded), water (45 mL), conc. H₂SO₄ (9.9 mL), 1.25 g of NaCl and methanol (12.5 mL) were added to the organic phase. After stirring for 1 hour the phases were separated and the aqueous phase was discarded, then a solution containing water (25 mL), methanol (5 mL) and 1.25 g of NaCl was added to the organic phase. The reaction mixture was stirred at 25-30°C for 30 minutes and the phases were separated, and again, a solution containing water (25 mL), methanol (5 mL) and 1.25 g of NaCl was added to the organic phase and the reaction mixture was stirred at 25-30°C for 30 minutes and the phases were separated and the aqueous phase was discarded. The organic phase was heated to reflux and solvents were distilled out at normal pressure to obtain pimavanserin hydrogen sulfate as an oily residue.

To residual pimavanserin hydrogen sulfate was added water (125 mL), methanol (12.5 mL) and 0.5 g of NaCl. After dissolution, the temperature of the reaction mixture was adjusted to 35-40°C, and then ethyl acetate (62 mL) was added and the mixture was stirred for 30 minutes at 35-40°C. The phases were then separated and the organic phase was discarded. To the aqueous phase (containing Pimavanserin hydrogen sulfate) was added ethyl acetate (62 mL) and the mixture was stirred at 35-40°C for 30 minutes. The phases were separated and the organic phase was discarded. Then, to the aqueous phase toluene (90 mL) was added. An 8% solution of NaHCO₃ was added dropwise to the aqueous/toluene solution until a pH value of 7.0-7.8 was reached. After stirring for 30 minutes at 35-40°C phases were separated, the aqueous phase was discarded and the organic phase (containing pimavanserin base) was used in next extraction. To organic phase was added water (37 mL) and n-heptane (50 mL) at 35-40°C. The reaction mixture was stirred for 30 minutes, the phases were separated and the aqueous phase was discarded. To the organic phase, n-heptane (100 mL) was added dropwise at 35-40°C. The obtained reaction mixture was stirred for 3 hours at 35-40°C, then cooled to 0-5°C over 5 hours, and stirred at 0-5°C for 4 hours. The product was filtered, washed first with a mixture of toluene:n-heptane and then with n-heptane.

The product was dried at 50-55°C to yield 6.73 g (56%) of pimavanserin base with a HPLC purity of 99.73% over three steps.

### Example 6: Preparation and purification of pimavanserin in "one pot" proceeding through pimavanserin acetate

Pimavanserin free base (4.8 g) was dissolved in solution of acetic acid (10 mL) and NaCl (1.0 g) in water (38 mL) and methanol (10 mL) mixture. The mixture was heated to 25-30°C and extracted with DCM (73 mL) over 1 h. After phase separation (water phase was discarded) the organic layer was extracted with 5% NaCl solution in 10% methanol. After stirring for 30 minutes, the phases were separated (water phase was discarded) and the solvents were removed under vacuum. The remaining oil of pimavanserin acetate was dissolved in water (50 mL) and methanol (5 mL) and 0.25 g of NaCl at 35-40°C. MTBE (50 mL) was then added and the mixture was stirred for 30 minutes at 35-40°C, the phases were then separated (organic phase was discarded). Toluene (36 mL) was then added to the aqueous phase and the pH was adjusted to 7.5 using 8% NaHCO₃ solution. After stirring for 30 minutes at 35-40°C the phases were separated (water phase was discarded). To the organic phase was added dropwise n-heptane (60 mL) at 35-40°C. The obtained reaction mixture was stirred for 3 h at 35-40°C, cooled to 0-5°C during 5 h, and stirred at 0-5°C for 4 h. The product was then filtered, and washed first with a mixture of toluene:n-heptane and then with n-heptane.

The product was dried at 50-55°C to yield 4.19 g (87%) of pimavanserin base with a HPLC purity of 99.85%.

### Example 7: Preparation and purification of pimavanserin in "one pot" proceeding through pimavanserin bitartrate

Pimavanserin free base (4.8 g) was dissolved in solution of tartaric acid (2.5 g) and NaCl (2.0 g) in water (38 mL) and methanol (10 mL) mixture. The mixture was heated to 30-35°C and extracted with DCM (73 mL) over 1 h. After phase separation (water phase was discarded) the organic layer was extracted with 5% NaCl solution in 10% methanol. After stirring for 30 minutes, the phases were separated (water phase was discarded) and the solvents were removed under vacuum. The remaining oil of pimavanserin bitartrate was dissolved in water (50 mL) and methanol (5 mL) and 0.25 g of NaCl at 35-40°C. MTBE (50 mL) was then added and mixture was stirred for 30 minutes at 35-40°C, the phases were then separated (organic phase was discarded). Toluene (36 mL) was then added and the pH was adjusted to 7.5 using 8% NaHCO₃ solution. After stirring for 30 minutes at 35-40°C the phases were separated (water phase was discarded). To the organic phase was added added dropwise n-heptane (60 mL) at 35-40°C. The obtained reaction mixture was stirred for 3 h at 35-40°C, cooled to 0-5°C during 5 h, and stirred at 0-5°C for 4 h. The product was then filtered, and washed first with a mixture of toluene:n-heptane and then with n-heptane.

The product was dried at 50-55°C to yield 4.23 g (88%) of pimavanserin base (PMV base) with a HPLC purity of 99.86%.

### Example 8: Purification of 1-[4-(2-methylpropyloxy)phenyllmethanamine acetate

1-[4-(2-methylpropyloxy)phenyl]methanamine acetate [PMV-4 acetate] (50 g) was suspended in a mixture of methanol (50 mL) and MTBE (50 mL). The reactor content was stirred while heating to 50-60°C. After full dissolution of PMV-4 acetate, MTBE 300 mL was added dropwise over 60 min and the mixture was stirred at its boiling point for an additional hour. The mixture was cooled to 0-5°C over 5 hours, then stirred at 0-5°C for 3 hours. The product was filtered off and washed with cold MTBE (100 mL). The product was then dried in tray dryer at 35-40°C for 6 h. Yield: 45.0 g (90%).

### Example 9: Conversion of 1-[4-(2-methylpropyloxy)phenyl]methanamine acetate to 1-[4-(2-methylpropyloxy)phenyl]methanamine

1-[4-(2-methylpropyloxy)phenyl]methanamine acetate (27 g, 0.11 mol) was added to 15% NaOH (135 mL) and dichloromethane (405 mL). The reactor content was stirred for 1 hour at 20-25°C and the aqueous and organic phases were separated. The organic layer was washed with 10% NaCl (135 mL) and then concentrated via distillation (at atmospheric pressure) to a yellow residue. The residual 1-[4-(2-methylpropyloxy)phenyl]methanamine was dissolved in dichloromethane (600 mL) and triethylamine (16 mL, 0.12 mol) was added. The solution was then transferred to a storage container (e.g. a dropping funnel) for further use.

## Claims

1. A process for the preparation of pimavanserin comprising:
(i) providing an acid addition salt solution of pimavanserin in an organic solvent;
(ii) washing the solution provided in step (i) with an alcohol/water/acid mixture;
(iii) dissolving the acid addition salt of pimavanserin in an aqueous solvent to form an aqueous solution;
(iv) washing the aqueous solution obtained in step (iii) with an organic solvent;
(v) adding a base to the washed aqueous solution to form pimavanserin.

2. A process for the preparation of an acid addition salt of pimavanserin comprising:
(i) providing an acid addition salt solution of pimavanserin in an organic solvent;
(ii) washing the solution provided in step (i) with an alcohol/water/acid mixture;
(iii) dissolving the acid addition salt of pimavanserin in an aqueous solvent to form an aqueous solution;
(iv) washing the aqueous solution obtained in step (iii) with an organic solvent;
(v) adding a base to the washed aqueous solution to form pimavanserin; and
(vi) converting pimavanserin into an acid addition salt of pimavanserin

3. A process as claimed in claim 2, wherein in step (vi) pimavanserin is converted into pimavanserin hemitartrate.

4. A process as claimed in claim 3, wherein prior to converting pimavanserin into pimavanserin hemitartrate in step (vi), the process further comprises the step of dissolving pimavanserin in a solvent to form a solution and adding a seed of pimavanserin hemitartrate form C thereto.

5. A process as claimed in claim 4, wherein pimavanserin is dissolved in a mixture of a solvent selected from acetone and methyl ethyl ketone; and a solvent selected from methyl-t-butyl ether, THF and heptane.

6. A process as claimed in claim 1 or any one of claims 2 to 5, wherein in step (iv) the organic solvent is selected from methyl-t-butyl ether, Me-THF, pentane, hexane, heptane, cyclohexane, methoxycyclohexane, diethyl ether, diisopropyl ether, ethyl acetate, isopropyl acetate, butyl acetate, methyl ethyl ketone, cyclopentyl methyl ether, toluene or xylene.

7. A process as claimed in claim 1 or any one of claims 2 to 6, wherein the base is a metal carbonate or a metal hydroxide.

8. A process as claimed in claim 6 or 7, wherein the acid addition salt of pimavanserin provided in step (i) of the process is formed by treating pimavanserin with a mineral acid or an organic acid.

9. A process as claimed in claim 8, wherein the pimavanserin obtained in step (v) of the process is recrystallised from a mixture of a solvent and an anti-solvent, wherein the solvent is selected from toluene, xylene, methyl-t-butyl ether, ethyl acetate, isopropyl acetate, Me-THF or cyclopentyl methyl ether; and the anti-solvent is selected from pentane, hexane, heptane, cyclohexane, methoxycyclohexane and petroleum ether.

10. A process as claimed in any preceding claim, wherein the process comprises an additional step, wherein 1-[4-(2-methylpropyloxy)phenyl]methanamine acetate is purified by recrystallisation, and the recrystallised 1-[4-(2-methylpropyloxy)phenyl]methanamine acetate is used to provide the acid addition salt of pimavanserin.

11. A process as claimed in claim 10, wherein the 1-[4-(2-methylpropyloxy)phenyl]methanamine acetate is recrystallised from an alcoholic solvent, or a mixture of an alcoholic solvent and non-alcoholic solvent.

12. A process for preparing a dosage form comprising pimavanserin, comprising the steps of preparing pimavanserin as claimed in claim 1 or any one of claims 6 to 11 and combining pimavanserin with one or more pharmaceutically acceptable excipients.

13. A process for preparing a dosage form comprising an acid addition salt of pimavanserin, comprising the steps of preparing an acid addition salt of pimavanserin as claimed in any one of claims 2 to 11 and combining the acid addition salt of pimavanserin with one or more pharmaceutically acceptable excipients.

## Patentansprüche

1. Verfahren zur Herstellung von Pimavanserin, umfassend:
(i) Bereitstellen einer Säureadditionssalzlösung von Pimavanserin in einem organischen Lösungsmittel;
(ii) Waschen der in Schritt (i) bereitgestellten Lösung mit einem Alkohol/Wasser/Säure-Gemisch;
(iii) Auflösen des Säureadditionssalzes von Pimavanserin in einem wässrigen Lösungsmittel, um eine wässrige Lösung zu bilden;
(iv) Waschen der in Schritt (iii) erhaltenen wässrigen Lösung mit einem organischen Lösungsmittel;
(v) Zugeben einer Base zu der gewaschenen wässrigen Lösung, um Pimavanserin zu bilden.

2. Verfahren zur Herstellung eines Säureadditionssalzes von Pimavanserin, umfassend:
(i) Bereitstellen einer Säureadditionssalzlösung von Pimavanserin in einem organischen Lösungsmittel;
(ii) Waschen der in Schritt (i) bereitgestellten Lösung mit einem Alkohol/Wasser/Säure-Gemisch;
(iii) Auflösen des Säureadditionssalzes von Pimavanserin in einem wässrigen Lösungsmittel, um eine wässrige Lösung zu bilden;
(iv) Waschen der in Schritt (iii) erhaltenen wässrigen Lösung mit einem organischen Lösungsmittel;
(v) Zugeben einer Base zu der gewaschenen wässrigen Lösung, um Pimavanserin zu bilden; und
(vi) Umwandeln von Pimavanserin in ein Säureadditionssalz von Pimavanserin.

3. Verfahren nach Anspruch 2, wobei in Schritt (vi) Pimavanserin in Pimavanserin-Hemitartrat umgewandelt wird.

4. Verfahren nach Anspruch 3, wobei das Verfahren vor der Umwandlung von Pimavanserin in Pimavanserin-Hemitartrat in Schritt (vi) ferner den Schritt des Auflösens von Pimavanserin in einem Lösungsmittel, um eine Lösung zu bilden, und das Zugeben eines Keims von Pimavanserin-Hemitartrat der Form C zu dieser umfasst.

5. Verfahren nach Anspruch 4, wobei Pimavanserin in einem Gemisch aus einem Lösungsmittel, ausgewählt aus Aceton und Methylethylketon, und einem Lösungsmittel, ausgewählt aus Methyl-*tert*-butylether, THF und Heptan, aufgelöst wird.

6. Verfahren nach Anspruch 1 oder einem der Ansprüche 2 bis 5, wobei in Schritt (iv) das organische Lösungsmittel aus Methyl-*tert*-butylether, Me-THF, Pentan, Hexan, Heptan, Cyclohexan, Methoxycyclohexan, Diethylether, Diisopropylether, Ethylacetat, Isopropylacetat, Butylacetat, Methylethylketon, Cyclopentylmethylether, Toluol oder Xylol ausgewählt wird.

7. Verfahren nach Anspruch 1 oder einem der Ansprüche 2 bis 6, wobei die Base ein Metallcarbonat oder ein Metallhydroxid ist.

8. Verfahren nach Anspruch 6 oder 7, wobei das in Schritt (i) des Verfahrens bereitgestellte Säureadditionssalz von Pimavanserin durch Behandeln von Pimavanserin mit einer Mineralsäure oder einer organischen Säure gebildet wird.

9. Verfahren nach Anspruch 8, wobei das in Schritt (v) des Verfahrens erhaltene Pimavanserin aus einem Gemisch aus einem Lösungsmittel und einem Antilösungsmittel umkristallisiert wird, wobei das Lösungsmittel aus Toluol, Xylol, Methyl-*tert*-butylether, Ethylacetat, Isopropylacetat, Me-THF oder Cyclopentylmethylether ausgewählt wird; und das Antilösungsmittel aus Pentan, Hexan, Heptan, Cyclohexan, Methoxycyclohexan und Petrolether ausgewählt wird.

10. Verfahren nach einem der vorhergehenden Ansprüche, wobei das Verfahren einen zusätzlichen Schritt umfasst, wobei 1-[4-(2-Methylpropyloxy)phenyl]methanaminacetat durch Umkristallisation gereinigt wird und das umkristallisierte 1-[4-(2-Methylpropyloxy)phenyl]methanaminacetat verwendet wird, um das Säureadditionssalz von Pimavanserin bereitzustellen.

11. Verfahren nach Anspruch 10, wobei das 1-[4-(2-Methylpropyloxy)phenyl]methanaminacetat aus einem alkoholischen Lösungsmittel oder einem Gemisch aus einem alkoholischen Lösungsmittel und einem nichtalkoholischen Lösungsmittel umkristallisiert wird.

12. Verfahren zur Herstellung einer Pimavanserin umfassenden Darreichungsform, umfassend die Schritte des Herstellens von Pimavanserin nach Anspruch 1 oder einem der Ansprüche 6 bis 11 und des Kombinierens von Pimavanserin mit einem oder mehreren pharmazeutisch annehmbaren Hilfsstoffen.

13. Verfahren zur Herstellung einer ein Säureadditionssalz von Pimavanserin umfassenden Darreichungsform, umfassend die Schritte des Herstellens eines Säureadditionssalzes von Pimavanserin nach einem der Ansprüche 2 bis 11 und des Kombinierens des Säureadditionssalzes von Pimavanserin mit einem oder mehreren pharmazeutisch annehmbaren Hilfsstoffen.

## Revendications

1. Procédé de préparation de pimavansérine comprenant:
(i) fournir une solution de sel d'addition avec un acide de pimavansérine dans un solvant organique;
(ii) laver la solution fournie dans l'étape (i) avec un mélange alcool/eau/acide;
(iii) dissoudre le sel d'addition avec un acide de pimavansérine dans un solvant aqueux pour former une solution aqueuse;
(iv) laver la solution aqueuse obtenue dans l'étape (iii) avec un solvant organique;
(v) ajouter une base à la solution aqueuse lavée pour former la pimava nsérine.

2. Procédé de préparation d'un sel d'addition avec un acide de pimavansérine comprenant:
(i) fournir une solution de sel d'addition avec un acide de pimavansérine dans un solvant organique;
(ii) laver la solution fournie dans l'étape (i) avec un mélange alcool/eau/acide;
(iii) dissoudre le sel d'addition avec un acide de pimavansérine dans un solvant aqueux pour former une solution aqueuse;
(iv) laver la solution aqueuse obtenue dans l'étape (iii) avec un solvant organique;
(v) ajouter une base à la solution aqueuse lavée pour former la pimavansérine; et
(vi) convertir la pimavansérine en un sel d'addition avec un acide de pimavansérine.

3. Procédé selon la revendication 2, dans lequel, dans l'étape (vi), la pimavansérine est convertie en hémitartrate de pimavansérine.

4. Procédé selon la revendication 3, dans lequel, avant de convertir la pimavansérine en hémitartrate de pimavansérine dans l'étape (vi), le procédé comprend en outre l'étape consistant à dissoudre la pimavansérine dans un solvant pour former une solution et à y ajouter un germe d'hémitartrate de pimavansérine forme C.

5. Procédé selon la revendication 4, dans lequel la pimavansérine est dissoute dans un mélange d'un solvant choisi parmi l'acétone et la méthyl éthyl cétone et d'un solvant choisi parmi le méthyl t-butyl éther, le THF et l'heptane.

6. Procédé selon la revendication 1 ou l'une quelconque des revendications 2 à 5, dans lequel, dans l'étape (iv), le solvant organique est choisi parmi le méthyl t-butyl éther, le Me-THF, le pentane, l'hexane, l'heptane, le cyclohexane, le méthoxycyclohexane, l'éther diéthylique, l'éther diisopropylique, l'acétate d'éthyle, l'acétate d'isopropyle, l'acétate de butyle, la méthyl éthyl cétone, le cyclopentyl méthyl éther, le toluène ou le xylène.

7. Procédé selon la revendication 1 ou l'une quelconque des revendications 2 à 6, dans lequel la base est un carbonate métallique ou un hydroxyde métallique.

8. Procédé selon l'une des revendications 6 ou 7, dans lequel le sel d'addition avec un acide de pimavansérine fourni dans l'étape (i) du procédé est formé par traitement de pimavansérine avec un acide minéral ou un acide organique.

9. Procédé selon la revendication 8, dans lequel la pimavansérine obtenue dans l'étape (v) du procédé est recristallisée à partir d'un mélange d'un solvant et d'un anti-solvant, dans lequel le solvant est choisi parmi le toluène, le xylène, le méthyl t-butyl éther, l'acétate d'éthyle, l'acétate d'isopropyle, le Me-THF ou le cyclopentyl méthyl éther, et l'anti-solvant est choisi parmi le pentane, l'hexane, l'heptane, le cyclohexane, le méthoxycyclohexane et l'éther de pétrole.

10. Procédé selon l'une quelconque des revendications précédentes, dans lequel le procédé comprend une étape supplémentaire dans laquelle l'acétate de 1-[4-(2-méthylpropyloxy)phényl]méthanamine est purifié par recristallisation, et l'acétate de 1-[4-(2-méthylpropyloxy)phényl]méthanamine recristallisé est utilisé pour fournir le sel d'addition avec un acide de pimavansérine.

11. Procédé selon la revendication 10, dans lequel l'acétate de 1-[4-(2-méthylpropyloxy)phényl]méthanamine est recristallisé à partir d'un solvant alcoolique ou d'un mélange d'un solvant alcoolique et d'un solvant non alcoolique.

12. Procédé de préparation d'une forme posologique comprenant de la pimavansérine, comprenant les étapes consistant à préparer la pimavansérine selon la revendication 1 ou l'une quelconque des revendications 6 à 11 et à combiner la pimavansérine avec un ou plusieurs excipients pharmaceutiquement acceptables.

13. Procédé de préparation d'une forme posologique comprenant un sel d'addition avec un acide de pimavansérine, comprenant les étapes consistant à préparer un sel d'addition avec un acide de pimavansérine selon l'une quelconque des revendications 2 à 11 et à combiner le sel d'addition avec un acide de pimavansérine avec un ou plusieurs excipients pharmaceutiquement acceptables.
